# EUROPEAN PATENT APPLICATION

(11) **EP 0 609 518 A1**
(43) Date of publication of application: **10.08.1994**
(21) Application number: 93119628.1
(22) Date of filing: 06.12.1993
(51) Int. Cl.: C07D 213/75, C07D 213/89, A61K 31/44

(54) **Optically active aminopyridine derivative and use thereof**

(30) Priority: 07.12.1992 JP 327013/92
(71) Applicant: THE GREEN CROSS CORPORATION, Osaka-shi Osaka 541 (JP)
(72) Inventor: Eda, Masahiro, c/o The Green Cross Corp., Hirakata-shi, Osaka 573 (JP); Okada, Takehiro, c/o The Green Cross Corp., Hirakata-shi, Osaka 573 (JP); Ono, Shinichiro, c/o The Green Cross Corp., Hirakata-shi, Osaka 573 (JP); Takemoto, Tadahiro, c/o The Green Cross Corp., Hirakata-shi, Osaka 573 (JP); Matsuno, Sumio, c/o The Green Cross Corp., Hirakata-shi, Osaka 573 (JP); Goda, Maki, c/o The Green Cross Corp., Hirakata-shi, Osaka 573 (JP); Ebisu, Hajime, c/o The Green Cross Corp., Hirakata-shi, Osaka 573 (JP); Nakamura, Norifumi, c/o The Green Cross Corp., Hirakata-shi, Osaka 573 (JP)
(74) Representative: VOSSIUS & PARTNER

(57) **Abstract**

A (+) antipode of an aminopyridine derivative, which is represented by the formula (I) 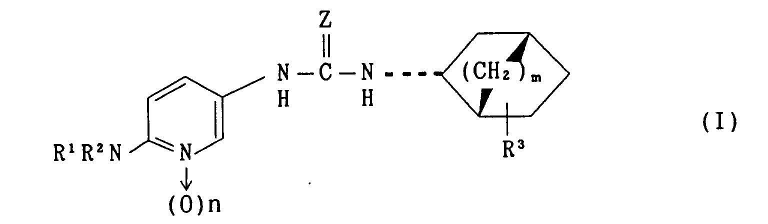
wherein eacy symbol is as defined in the Specification, a salt thereof, and the use thereof as an agent for circulatory diseases. The compound of the invention is extremely low toxic and shows strong and long-lasting pharmacological action such as vasodepressor action. When used as a preventive or therapeutic agent for hypertension, in particular, the compound affords stable antihypertensive action even at diminished administration frequencies. Also, it can be used as a lipometabolism-improving agent.

## Description

The present invention relates to a (+) antipode of an aminopyridine compound, which is useful as an agent for circulatory diseases, particularly as an antihypertensive agent, and to an agent for circulatory diseases containing the antipode as an active ingredient.

Various compounds have been marketed and developed as agents for circulatory diseases, particularly as therapeutic agents for hypertension. Recently, the possibility of treating circulatory diseases based on potassium channel opening (which is a new mechanism) has been suggested, and various studies based on this theory have been done.

The representative compounds for the potassium channel openers are pinacidil [N''-cyano-N-4-pyridyl-N'-(1,2,2-trimethylpropyl)guanidine] having an N-pyridine-N'-cyanoguanidine skeleton and cromakalim [(+,-)-6-cyano-3,4-dihydro-2,2-dimethyl-trans-4-(2-oxo-1-pyrrolidinyl)-2H-benzo(b)pyran-3-ol] having a benzopyran skeleton.

However, neither pinacidil nor cromakalim necessarily exhibits sufficient pharmacological action, and side effects have not been reduced to a satisfactory level. In particular, pinacidil has the side effect of body fluid retention, causing problems of edema, vascular headache, cardiopalmus (palpitation), and so on.

On the other hand, studies of new compounds capable of solving such problems and exhibiting improved pharmacological effects are being done. Examples of such compounds include N-alkyl-N'-pyridylthiourea and N-alkyl-N'-pyridyl-N'-cyanoguanidine (US Patent No. 4057636, US RE 31244), and N-substituted N-arylthiourea and N-substituted N-aryl-N'-cyanoguanidine (EP 354553, EP 392802). Also, EP 503627 discloses aminopyridine derivatives.

There is, however, still a demand for a compound having improved pharmacological effects and reducted side effects.

It has been found that (+) antipodes of a group of compounds from the aminopyridine derivatives disclosed in EP 503627 have superior properties in terms of their pharmacological effects and reduced side effects.

Accordingly, the present invention relates to a (+) antipode of an aminopyridine derivative, which is represented by the formula (I) 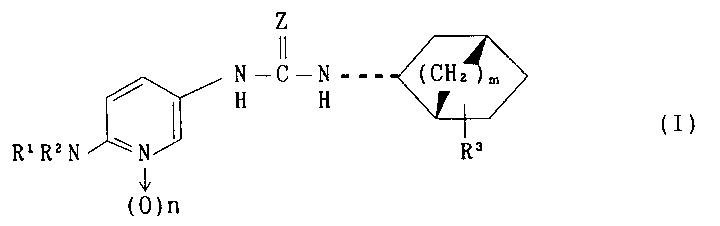
wherein R¹ and R² may be the same or different and each is hydrogen, alkyl having 1 to 6 carbon atoms, or an amino-protecting group, R³ is hydrogen or alkyl having 1 to 6 carbon atoms, Z is S or NCN, m is 1 or 2, and n is 0 or 1 [hereinafter also referred to as Compound (I)], or an acid addition salt thereof.

The present invention also relates to an agent for circulatory diseases containing Compound (I) or an acid addition salt thereof as an active ingredient.

As regards R¹ and R² in the formula (I), the alkyl having 1 to 6 carbon atoms may be straight or branched and is exemplified by methyl, ethyl, propyl, butyl, pentyl, hexyl, t-butyl, and the like, and examples of the amino-protecting group include amino-protecting groups known per se, such as acyl (e.g. acetyl, formyl, benzoyl, t-butoxycarbonyl), benzyl, and phthalimide. The preferable R¹ and R² groups are alkyls having 1 to 3 carbon atoms.

As regards R³, the alkyl having 1 to 6 carbon atoms may be straight or branched, like the alkyl for R¹ and R², and is exemplified likewise. Of those, preferred is an alkyl having 1 to 3 carbon atoms.

In the present specification, (+) antipode means an enantiomer which rotates to the right on the vibration plane of the linearly polarized light of the sodium D-line.

The typical synthesis methods of the Compound (I) of the present invention are as follows. 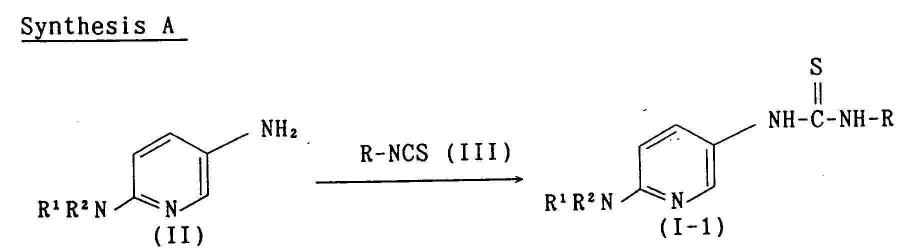
wherein R is a group of the formula 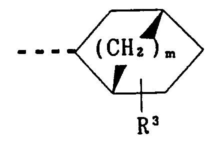
(where R³ and m are as defined above), and R¹ and R² are as defined above.

A compound of the formula (II) is reacted with a compound of the formula (III). The reaction is generally carried out in a solvent. The solvent may be any insofar as it does not adversely affect the reaction and is exemplified by a protic polar solvent such as methanol and ethanol, an aprotic polar solvent such as ethers (e.g. diethyl ether, tetrahydrofuran, dioxane), halogenated hydrocarbons (e.g. dichloromethane, dichloroethane, chloroform, carbon tetrachloride), aromatic hydrocarbons (e.g. benzene, toluene, xylen), heterocyclic compounds (e.g. pyridine, piperidine), dimethylformamide (DMF), and dimethylsulfoxide.

The ratio of the compound (III) is suitably from about 0.9 molar to 5 molar proportion, preferably from about 1 molar to 3 molar proportion, to 1 molar proportion of the compound (II).

The reaction proceeds at a temperature between 10°C and 80°C, preferably at room temperature (i.e. 15-25°C) for about 1 to 200 hours.

The compound (III) can be prepared, for example, according to the method disclosed in M. L. Moore et al, Org. Synth., III, *599* (1955), as in the following. 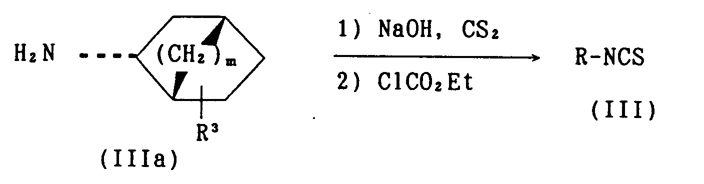
wherein R, R³, and m are as defined above.

The amine derivative (IIIa) (which is the starting material) can be obtained by converting the carboxylic acid derivative as described in T. Poll et al, Tetrahedron. Lett., *30*, 5595 (1989) to an amine derivative by the method disclosed in Japanese Patent Unexamined Publication No. 287794/1988 or a method analogous thereto. 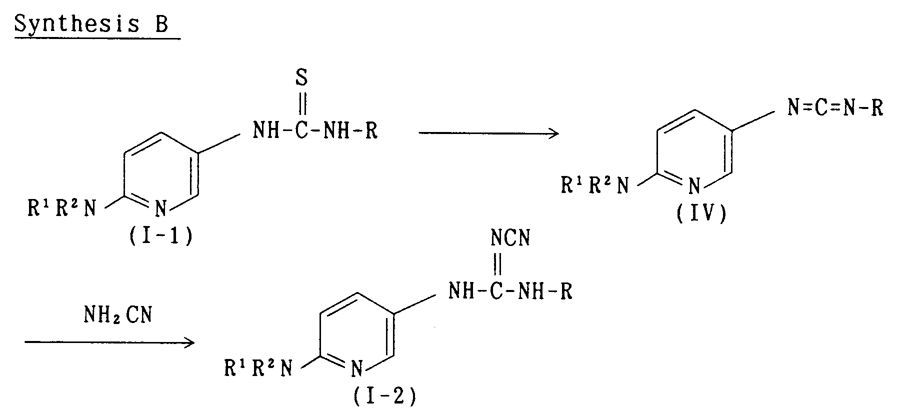
wherein R, R¹, and R² are as defined above.

### [Step 1]

Step 1 is a reaction for obtaining a carbodiimide compound of the formula (IV) from the thiourea compound of the formula (I-1). The reaction can be carried out by a conventional method for synthesizing a carbodiimide compound from a thiourea compound. For example, a thiourea compound is reacted with a metal oxide such as mercury oxide or lead oxide in the presence of sulfur where necessary, or a thiourea compound and triphenylphosphine are reacted in the presence of carbon tetrachloride and triethylamine. The reaction is usually carried out in a solvent. The solvent may be any insofar as it does not adversely affect the reaction and is exemplified by halogenated hydrocarbons such as dichloromethane, dichloroethane, chloroform, and carbon tetrachloride, ethers such as ether and dioxane, aromatic hydrocarbons such as benzene, toluene, and xylen, alcohols such as ethanol, and pyridine.

The ratio of metal oxide or triphenylphosphine is about 0.9 molar to 2 molar proportion, preferably from about 1 molar to 1.2 molar proportion, to 1 molar proportion of the thiourea compound (I-1). The reaction proceeds at a temperature from room temperature (i.e. 15-25°C) to the boiling point of the solvent, preferably from 30°C to 60°C for about 10 to 100 hours.

### [Step 2]

Step 2 is a reaction for obtaining a compound of the formula (I-2) by reacting the carbodiimide compound (IV) obtained above with cyanamide. The reaction is usually carried out in a solvent. The solvent may be any insofar as it does not adversely affect the reaction and is exemplified by halogenated hydrocarbons such as dichloromethane, dichloroethane, chloroform, and carbon tetrachloride, ethers such as ether and dioxane, alcohols such as ethanol, and pyridine.

The ratio of cyanamide is from about 0.9 molar to 2 molar proportion, preferably from about 1 molar to 1.2 molar proportion, to 1 molar proportion of the carbodiimide compound (IV). The reaction is carried out in the presence of a basic substance such as diisopropylethylamine at 10-30°C for about 1 to 50 hours. 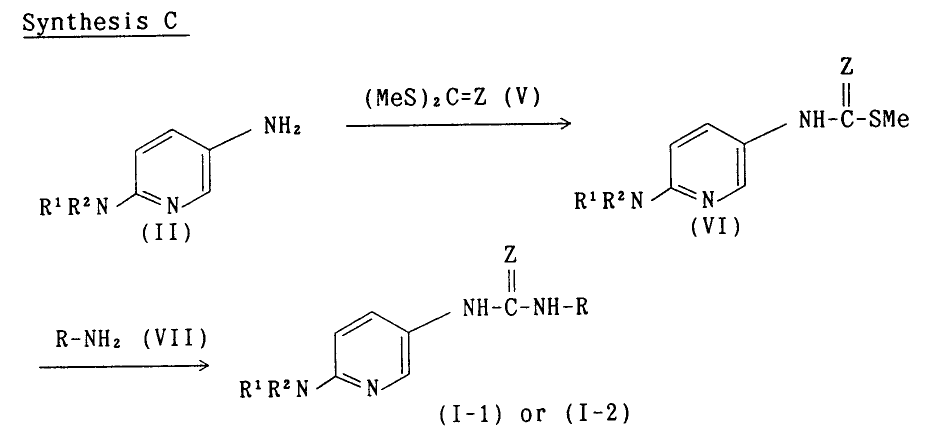
wherein Z, R, R¹, and R² are as defined above.

### [Step 1]

Step 1 is a reaction for obtaining a compound of the formula (VI) by reacting a compound of the formula (II) with a compound of the formula (V). The reaction is usually carried out in a solvent. The solvent may be any insofar as it does not adversely affect the reaction and is exemplified by the solvent recited in Synthesis B, with preference given to a polar solvent such as pyridine, dimethylformamide (DMF), or ethanol.

The ratio of the compound (V) is from about 0.9 molar to 5 molar proportion, preferably from about 1 molar to 2 molar proportion, to 1 molar proportion of the compound (II).

The reaction proceeds at a temperature of about 10-80°C, preferably at about room temperature (i.e. 15-25°C) for about 1 to 200 hours.

### [Step 2]

Step 2 is a reaction for obtaining a compound of the formula (I-1) or (I-2) by reacting the compound (VI) obtained above with a compound of the formula (VII). The reaction usually proceeds in or without a solvent. When a solvent is used, the solvent may be any insofar as it does not adversely affect the reaction and is preferably exemplified by a polar solvent such as pyridine, dimethylformamide (DMF), and ethanol.

The ratio of the compound (VII) is from about 0.9 molar to 10 molar proportion, preferably from about 1 molar to 3 molar proportion, to 1 molar proportion of the compound (VI).

The reaction is carried out at 10-90°C for about 1 to 100 hours.

### Synthesis D

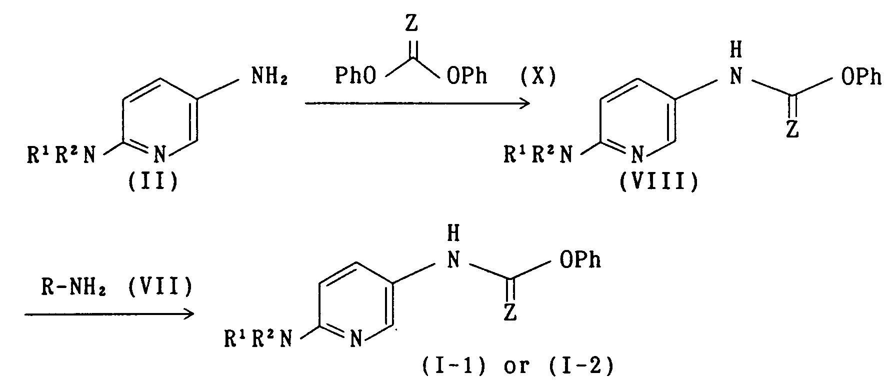

wherein Z, R, R¹, and R² are as defined above.

### [Step 1]

Step 1 is a reaction for obtaining a compound of the formula (VIII) by reacting a compound of the formula (II) and a compound of the formula (X). The compound (X) can be prepared by the method described in the literature [Lee Webb, R. and Labaw, C.S., Heterocyclic Chem., 1205, *19* (1982)]. The reaction is usually carried out in a solvent. The solvent may be any insofar as it does not adversely affect the reaction and may be any of those exemplified in Synthesis A. Particularly preferable is a polar solvent such as acetonitrile, tetrahydrofuran, or isopropyl alcohol. The ratio of the compound (X) is about 0.9 molar to 2 molar proportion, preferably from about 1 molar to 1.2 molar proportion, to 1 molar proportion of the compound (II). The reaction proceeds at a temperature between about 10°C and 80°C, preferably at about room temperature (i.e. 15-25°C) for about 10 minutes to 2 hours.

### [Step 2]

Step 2 is a reaction for obtaining a compound of the formula (I-1) or (I-2) by reacting the compound (VIII) obtained above with a compound of the formula (VII). The reaction is usually carried out in a solvent. The solvent may be any insofar as it does not adversely affect the reaction and may be any of those exemplified in Synthesis A. Preferred is a polar solvent such as ethyl acetate, tetrahydrofuran, or isopropyl alcohol. The ratio of the compound (VII) is about 0.9 molar to 2 molar proportion, preferably from about 1 molar to 1.2 molar proportion, to 1 molar proportion of the compound (VIII). The reaction proceeds at a temperature between about 10°C and 100°C, preferably at about 80°C for about 1 to 48 hours. 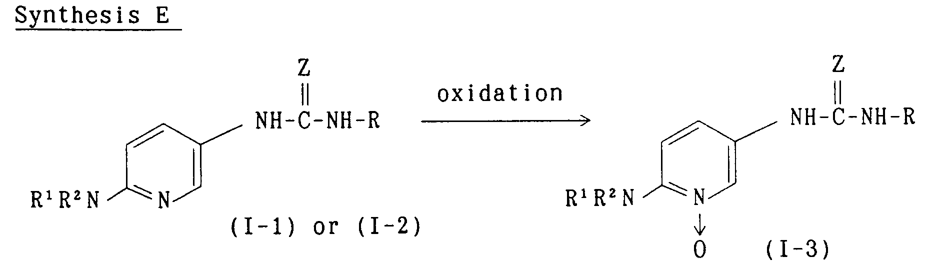
wherein Z, R, R¹, and R² are as defined above.

The compound of the formula (I-3) can be obtained by subjecting the compound of the formula (I-1) or (I-2) to an oxidation reaction. The oxidation reaction proceeds in the presence of an oxidizing agent. The reaction is usually carried out in a solvent. The solvent may be any insofar as it does not adversely affect the reaction and is exemplified by halogenated hydrocarbons such as dichloromethane, dichloroethane, chloroform, and carbon tetrachloride, alcohols such as methanol and ethanol, aromatic hydrocarbons such as benzene, toluene, and xylen, and fatty acids such as acetic acid and propionic acid. Examples of the oxidizing agent to be used in the reaction include peracids such as peracetic acid, perbenzoic acid, and m-chloroperbenzoic acid, and hydrogen peroxide.

The ratio of the oxidizing agent is from about 0.9 molar to 2 molar proportion, preferably from about 1 molar to 1.2 molar proportion, to 1 molar proportion of the compound (I-1) or (I-2).

The reaction proceeds at a temperature from ice-cooling to under heating, preferably from about ice-cooling to room temperature (i.e. 15-25°C) for about 1 to 10 hours.

As regards the formula (I), a compound wherein R¹ and/or R² are(is) (an) amino-protecting group(s) can be converted to a compound wherein R¹ and/or R² are(is) hydrogen(s) by deprotection by a method known per se. The elimination of the protecting group varies depending on the kind of the protecting group, and may be performed by a method comprising treatment with an acid (e.g. hydrochloric acid) or Ingemans method using hydrazine, which is to be selected as appropriate.

The preferable compound is hydrochloride of (+)-N-(6-amino-3-pyridyl)-N''-cyano-N'-[(1S,2R,4R)-2-norbornyl]guanidine.

The (+) antipode of an aminopyridine compound of the present invention can also be produced by optical resolution of the racemic mixture or racemic compound of aminopyridine, besides the aforementioned Syntheses A to E.

The novel Compound (I) of the present invention thus produced can be obtained at an optional purity by known separation and purification means employed as appropriate, such as concentration, extraction, chromatography, reprecipitation, and recrystallization.

Since the Compound (I) of the present invention has a basic group, it can be converted to an acid addition salt by a known means. The salt is subject to no particular limitation insofar as it is a pharmacologically acceptable, nontoxic salt, and is exemplified by salts with inorganic acid, such as hydrochloride, hydrobromide, phosphate, and sulfate, salts with organic acid, such as acetate, succinate, maleate, fumarate, malate, and tartrate, and the like.

The Compound (I) of the present invention and an acid addition salt thereof exhibit strong and long-lasting vasodepressor action, angiotelectasis action, coronary vasodilative action, cerebral vasodilative action, and so on, in mammals such as mouse, rat, rabbit, dog, cat, human, and so on, and are extremely low toxic. Accordingly, the Compound (I) of the present invention or an acid addition salt thereof are useful as a preventive and a therapeutic agent for circulatory diseases in, for example, humans, such as hypertension, ischemic heart diseases (e.g. angina pectoris, myocardinal infarction), disorders of cerebral or peripheral circulatory organs (e.g. cerebral infarction, transient ischemic attack), and so on.

Particularly, the Compound (I) and an acid addition salt thereof of the present invention are superior in potency and duration of the pharmacological action to the degree that a stable antihypertensive action can be attained with a few administrations (once or twice a day) when used, for example, for the prevention or treatment of hypertension.

Since the Compound (I) and an acid addition salt thereof of the present invention can greatly improve lipometabolism, it can be also used as a lipometabolism-improving agent such as a therapeutic agent for hyperlipidemia. Besides, it is expected to act as a relaxant for various smooth muscle organs (e.g. gastrointestinal tract, respiratory system, uterus).

When the Compound (I) or an acid addition salt thereof of the present invention is used as the above-mentioned pharmaceutical, it may be mixed with pharmacologically acceptable additives such as a carrier, excipient, diluent, and so on and formulated into a pharmaceutical composition in the form of a powder, granule, tablet, capsule, injection, or the like, which is administered orally or parenterally. Said preparation contains an effective amount of the Compound (I) or an acid addition salt thereof of the present invention. While the dose varies depending on administration route, symptom, body weight, and age of patient, it is generally 0.05-20 mg/kg body weight/day, preferably 0.1-4 mg/kg body weight/day in one to several times divided doses for oral administration to an adult with hypertension.

The present invention is more detailedly explained in the following by referring to Examples, to which the present invention is not limited.

### Example 1

### (1) Production of optically active (1S,2R,4R)-2-norbornyl-isothiocyanate

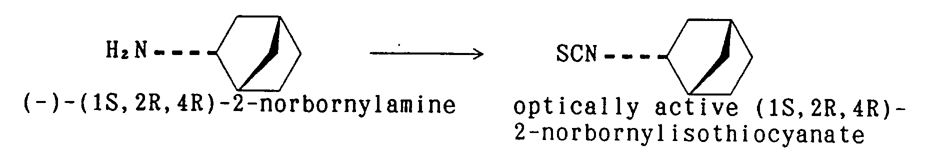

Sodium hydroxide (2.904 g) was dissolved in water (17 ml), and carbon disulfide (5.240 ml) was added thereto under ice-cooling. Gradual addition of (-)-(1S,2R,4R)-2-norbornylamine (8 g) thereto under ice-cooling resulted in precipitation of white crystals. After stirring the mixture at room temperature for 1 hour, it was stirred at 70°C for 1 hour to give a monolayer orange liquid. The liquid was allowed to cool to room temperature, and thereto was dropwise added ethyl chlorocarbonate (7.636 ml), followed by stirring at room temperature for 1 hour to give a two-layered yellow liquid. The oily substance of the bottom layer was taken out with a separatory funnel, suspended in a 20% aqueous solution of sodium hydroxide (20 ml), and vigorously stirred for 16 hours. Water (20 ml) was added thereto and the mixture was extracted with hexane. After drying over magnesium sulfate, the solvent was distilled away, and the resultant product was subjected to column purification using hexane to give a white powder of the objective optically active (1S,2R,4R)-2-norbornylisothiocyanate. Yield: 5.109 g (46.5%)

### (2) Production of optically active N-(6-amino-3-pyridyl)-N'-[(1S,2R,4R)-2-norbornyl]thiourea

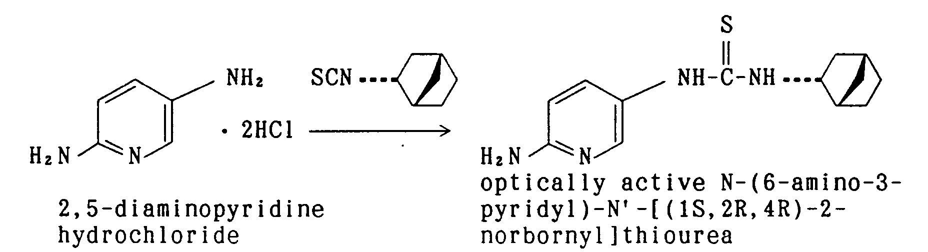

2,5-Diaminopyridine hydrochloride (4 g) was dissolved in dimethylformamide (DMF) (20 ml), and the optically active (1S,2R,4R)-2-norbornylisothiocyanate (3.655 g) as obtained in (1) above and then triethylamine (6.125 ml) were added thereto. The mixture was stirred at room temperature for 16 hours, added with a 5% aqueous solution of potassium carbonate (80 ml) and water (80 ml), and extracted with ethyl acetate. The solvent was distilled away, and the resultant product was purified by column chromatography (chloroform:methanol=10:1 by volume) and recrystallized from methylene chloride-methanol-diethyl ether to give a pale pink powder of the objective optically active N-(6-amino-3-pyridyl)-N'-[(1S,2R,4R)-2-norbornyl]thiourea. Yield: 3.402 g (59.0%) Melting point: 173.0-174.0°C.

¹H-NMR of the object compound is as follows.
¹H-NMR (DMSO): 0.7-1.7 (7H, m), 1.8-2.1 (1H, m), 2.1-2.25 (1H, m), 2.4-2.6 (1H, m), 4.2-4.45 (1H, m), 5.77 (2H, brs), 6.40 (1H, d, J=8.0Hz), 7.37 (1H, dd, J=2.0, 8.0Hz), 7.55 (1H, brs), 7.77 (1H, d, J=2.0Hz), 8.84 (1H, brs)
IR (KBr): 3500-3000, 2900, 2850, 1630, 1500, 1530

### (3) Production of optically active N-(6-amino-3-pyridyl)-N'-[(1S,2R,4R)-2-norbornyl]carbodiimide

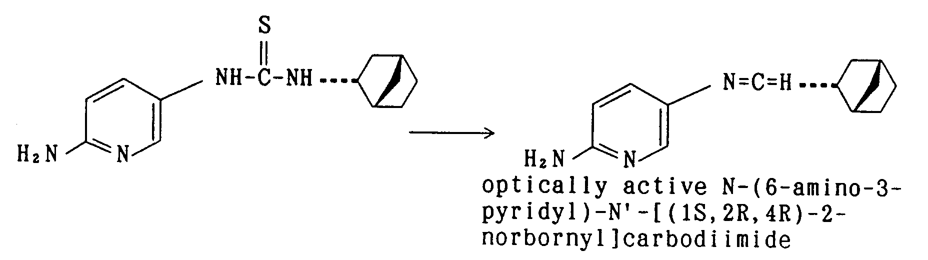

In a 1 liter pear-type flask equipped with Dimroth condenser and packed with blue silica gel was charged a solution of optically active N-(6-amino-3-pyridyl)-N'-[(1S,2R,4R)-2-norbornyl]thiourea (29.7 g, 113.2 mmol) as obtained in (2) above in ethanol (150 ml)-methylene chloride (150 ml), and mercury oxide (49.0 g, 226.4 mmol) and sulfur powder (1.82 g, 56.6 mmol) were added thereto. The mixture was stirred under reflux for 15 hours, added with mercury oxide (20.0 g, 92.3 mmol) and sulfur powder (500 mg, 15.6 mmol), and stirred under reflux for 2.5 hours. The insoluble matters were filtered off with celite, and the filtrate was concentrated to leave about 100 ml of ethanol to give an ethanol solution containing the object compound.

### (4) Production of (+)-N-(6-amino-3-pyridyl)-N''-cyano-N'-[(1S,2R,4R)-2-norbornyl]guanidine

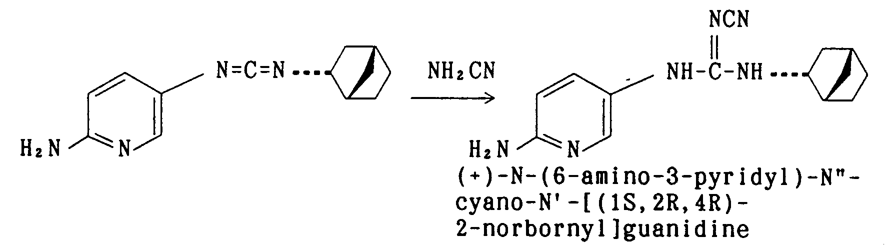

A solution of optically active N-(6-amino-3-pyridyl)-N'-[(1S,2R,4R)-2-norbornyl]carbodiimide (25.8 g, 113.2 mmol) as obtained in (3) above in ethanol (200 ml)-methylene chloride (250 ml) was charged in a 2 liter three-mouthed separate flask equipped with a nitrogen balloon. Cyanamide (5.23 g, 124.5 mmol) and diisopropylethylamine (9.9 ml, 56.6 mmol) were added thereto, and the mixture was stirred at room temperature for 18 hours. Then, cyanamide (2.5 g, 59.5 mmol) was added thereto, and the mixture was stirred for 72 hours. The precipitate was filtered off and recrystallized to give the object compound as white needle crystals. Yield: 22.5 g (74%) [α]_{D}=+12.0 (C=0.5 ethanol) Melting point 165-166°C, 184-185°C
¹H-NMR (DMSO): 0.9-1.6 (7H, m), 1.7-2.05 (1H, m), 2.05-2.2 (1H, brs), 2.3-2.45 (1H, m), 3.85-4.1 (1H, m), 5.91 (2H, s), 6.43 (1H, d, J=8.0Hz), 6.58 (1H, d, J=4.0Hz), 7.21 (1H, dd, J=2.0, 8.0Hz), 7.73 (1H, d, J=2.0Hz), 8.46 (1H, s)
IR (KBr): 3500-3000, 2900, 2850, 2150, 1600, 1500

### (5) Production of hydrochloride of (+)-N-(6-amino-3-pyridyl)-N''-cyano-N'-[(1S,2R,4R)-2-norbornyl]guanidine

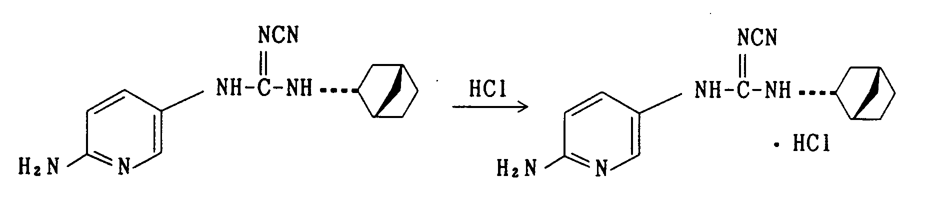

A solution of (+)-N-(6-amino-3-pyridyl)-N''-cyano-N'-[(1S,2R,4R)-2-norbornyl]guanidine (21.9 g, 81.0 mmol) in methylene chloride (250 ml)-methanol (100 ml) was charged in a 1 liter pear-type flask. 4.74N Hydrochrolic acid in ethanol (17.1 ml, 81.0 mmol) was dropwise added gradually. The mixture was stirred at room temperature for 1 hour, and the solvent was distilled away under reduced pressure. The precipitate was filtered off, dried in a vacuum pump at 78°C for 6 hours to give the objective compound as a white powder. Yield: 21.22 g (85%) [α]_{D}=+26.5 (C=0.5 ethanol)
¹H-NMR (DMSO): 0.9-1.7 (7H, m), 1.75-2.05 (1H, m), 2.05-2.25 (1H, m), 2.35-2.50 (1H, m), 3.9-4.2 (1H, m), 7.03 (1H, d, J=I0.0Hz), 7.44 (1H, d, J=6.0Hz), 7.84 (1H, d, J=10.0Hz), 7.98 (1H, s), 8.0-8.4 (2H, brs), 9.31 (1H, brs)
IR (KBr): 3600-2200, 2150

### Example 2

### (+)-N-(6-Amino-3-pyridyl)-N''-cyano-N'-[(1S,2R,4R)-2-norbornyl]guanidine was obtained by Synthesis C.

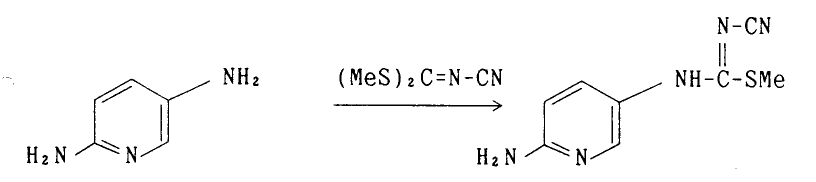

That is, 2,5-diaminopyridine and S,S'-dimethyl N-cyanothioiminocarbonate were reacted. 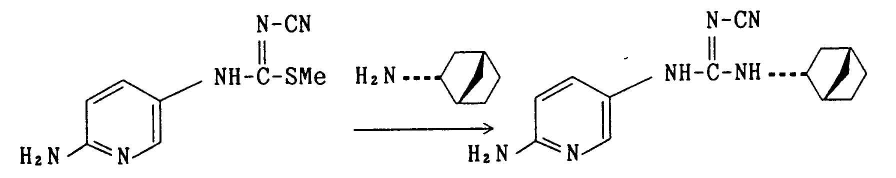
By reacting the obtained product with (-)-(1S,2R,4R)-2-norbornylamine, the object compound was obtained.

### Example 3

### According to Synthesis D, there was obtained (+)-N-(6-amino-3-pyridyl)-N''-cyano-N'-[(1S,2R,4R)-2-norbornyl]guanidine.

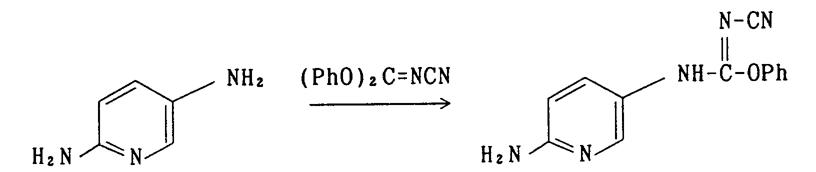

That is, 2,5-diaminopyridine was reacted with diphenylcyanocarbonimidate. 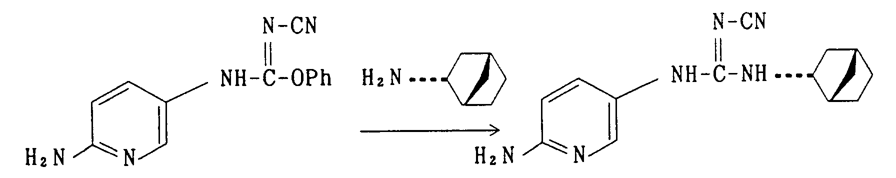
The obtained N-cyano-N'-3-(6-aminopyridyl)-O-phenylisourea was reacted with (-)-(1S,2R,4R)-2-norbornylamine to give the object compound (yield 68%).

### Experimental Example 1

### Suppression of aorta thoracica constriction

The aorta thoracica used was that of male Wistar rat.

A rat weighing about 300 g was sacrified, and incised at the chest to remove the aorta decendens. The removed aorta was placed in a nutritive solution (PSS: NaCl 117 mM, KCl 5.0 mM, CaCl₂ 1.5 mM, NaH₂PO₄ 1.0 mM, NaHCO₃ 25 mM, MgSO₄ · 7H₂O 1.2 mM, glucose 11.5 mM) to remove the fat tissues attached to the aorta, and cut helically into strips (10 mm long). The aorta strips were hung in PSS aerated with a mixed gas (oxygen: 95%, carbon dioxide: 5%) in Magnus bathes (20 ml) at 37°C and a load of 1g was applied, and a change in tension was recorded on a thermal stylus recorder via a converter.

Upon stabilization of the tension, the specimen was contracted with KCl (30 mM) twice for 20 minutes (washed each time and 20 minutes interval was taken), followed by washing. The drugs recited in Table 1 were cumulatively added to the blood vessel strips contracted with KCl (20 mM). The relaxation was taken as 0 prior to the addition of each drug, and taken as complete when the specimen was relaxed by the addition of papaverine (10-⁴ M). IC₅₀ is the concentration of the compounds when 50% relaxation of the strips was attained. The results are shown in Table 1.

**Table 1**

| Compound | IC₅₀ |
|---|---|
| hydrochloride of (+)-N-(6-amino-3-pyridyl)-N''-cyano-N'-[(1S,2R,4R)-2-norbornyl]guanidine | 8.8×10⁻⁸M |
| hydrochloride of N-(6-amino-3-pyridyl)-N''-cyano-N'-[(1S,2R,4R)-2-norbornyl]guanidine racemate | 4.8x10⁻⁷M |
| hydrochloride of (-)-N-(6-amino-3-pyridyl)-N''-cyano-N'-[(1S,2R,4R)-2-norbornyl]guanidine | 7.0×10⁻⁷M |
| Cromakalim | 9.4×10⁻⁸M |
| Pinacidil | 2.4×10⁻⁷M |

### Experiment 2

### Antihypertensive action

The compound (hydrochloride) of the present invention obtained in Example 1 was dissolved in brine, and forcibly administered (p.o.) to male spontaneously hypertensive rat at 3 mg/kg. The systolic pressure was measured with a programmable sphygmonanometer (PS-200A, manufactured by Riken Kaihatsu, Japan) by tail-cuff method [Ohkubo H et al. Proc. Natl. Acad. Sci. USA, *87*, 5153-5157 (1990)].

The vasodepressor action was 33% at 1 hour from the oral administration.

### Experiment 3

### Toxicity by single administration to rat

Administration of 450 mg/kg of the compound (hydrochloride) of the present invention did not result in a death case.

| Formulation Example 1 : Tablets | | |
|---|---|---|
| (1) | Compound (I) of the invention | 10 mg |
| (2) | Fine particle for direct compression No. 209 (manufactured by Fuji Kagaku Corp., Japan) | 46.6 mg |
| | magnesium aluminate metasilicate | 20% |
| | corn starch | 30% |
| | lactose | 50% |
| (3) | Crystalline cellulose | 24.0 mg |
| (4) | Carboxylmethyl cellulose · calcium | 4.0 mg |
| (5) | Magnesium stearate | 0.4 mg |

(1), (3), and (4) are previously passed through a 100-mesh sieve. (1), (3), (4), and (2) are respectively dried to a certain moisture content, and kneaded in a kneader at the above-mentioned weight ratio. (5) is then added to the uniform powder mixture and mixed for a short time (30 sec), and the powder mixture is compressed into tablets (pounder: 6.3 mm⌀, 6.0 mmR) of 85 mg per tablet.

The obtained tablets may be coated with an enteric film coating agent such as poly(vinyl acetal) diethylaminoacetate or an edible colorant conventionally employed, on demand.

| Formulation Example 2 : Capsules | | |
|---|---|---|
| (1) | Compound (1) of the invention | 50 g |
| (2) | Lactose | 935 g |
| (3) | Magnesium stearate | 15 g |

The above ingredients are weighed and uniformly mixed. The powder mixture is packed in hard gelatin capsules by 200 mg.

| Formulation Example 3 : Injections | | |
|---|---|---|
| (1) | Compound (1) of the invention · hydrochloride | 5 mg |
| (2) | Sucrose | 100 mg |
| (3) | Physiological saline | 10 ml |

A mixed solution of the above ingredients is filtered through a membrane filter, and further sterilized by filtration. Then, the filtrate is dispensed to a vial aseptically and nitrogen gas is charged, after which the vial is sealed to give an intravenous injection.

The compound of the present invention has extremely low toxicity, and has strong and long-lasting vasodepressor action, angiotelectasis action, coronary vasodilative action, cerebral vasodilative action, and so on. Hence, the compound of the invention is useful as an agent for the prophylaxis and treatment of circulatory diseases. In particular, stable antihypertensive action can be attained at a less administration frequency when the compound is used as an antihypertensive for the prophylaxis and treatment of hypertension.

In view of the superior action that the compound has for improving lipometabolism, its use as an agent for improving lipometabolism such as a therapeutic agent for hyperlipidemia as well as a relaxant for various smooth muscle organs is also expected.

## Claims

1. A (+) antipode of an aminopyridine derivative, which is represented by the formula (I) 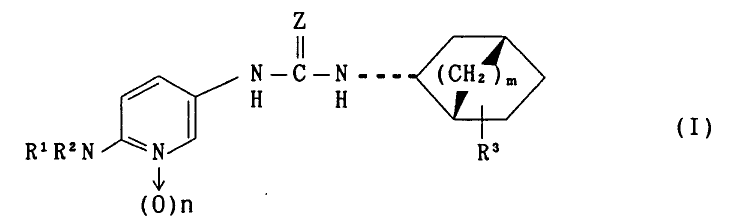 wherein R¹ and R² is the same or different and each is hydrogen, alkyl having 1 to 6 carbon atoms, or an amino-protecting group, R³ is hydrogen or alkyl having 1 to 6 carbon atoms, Z is S or NCN, m is 1 or 2, and n is 0 or 1, or an acid addition salt thereof.

2. The antipode of Claim 1, which is (+)-N-(6-amino-3-pyridyl)-N''-cyano-N'-[(1S,2R,4R)-2-norbornyl]guanidine, or an acid addition salt thereof.

3. A pharmaceutical composition comprising, as an active ingredient, a (+) antipode of an aminopyridine derivative, which is represented by the formula (I) 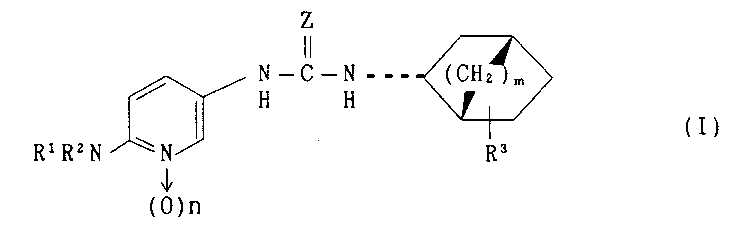 wherein R¹ and R² is the same or different and each is hydrogen, alkyl having 1 to 6 carbon atoms, or an amino-protecting group, R³ is hydrogen or alkyl having 1 to 6 carbon atoms, Z is S or NCN, m is 1 or 2, and n is 0 or 1, or an acid addition salt thereof, and a pharmacologically acceptable additive.

4. The composition of Claim 3, which comprises (+)-N-(6-amino-3-pyridyl)-N''-cyano-N'-[(1S,2R,4R)-2-norbornyl]guanidine, or an acid addition salt thereof.

5. Use of a compound as defined in claims 1 or 2 for the manufacture of a medicament for use as an antihypertensive.
